Europäisches Patentamt

European Patent Office

Office européen des brevets

(11) Publication number: **0 430 699 A2**

# EUROPEAN PATENT APPLICATION

(21) Application number: 90313021.9

(51) Int. Cl.5: **C07C 31/04**, C07C 29/86

(22) Date of filing: 30.11.90

(30) Priority: 01.12.89 GB 8927266

(43) Date of publication of application:
05.06.91 Bulletin 91/23

(84) Designated Contracting States:
AT BE CH DE DK ES FR GB GR IT LI LU NL SE
Bulletin 1

(71) Applicant: CSIR
Corporate Building, Scientia
Pretoria Transvaal Province(ZA)

(72) Inventor: Van Vuuren, David Steyn
c/o 100 Malan Street, Riviera
Pretoria, Transvaal(ZA)
Inventor: Mulder, Harko
c/o 48 Stinkhout Crescent, SE3
Vanderbijlpark, Transvaal(ZA)
Inventor: Nel, Hercules Francois
c/o 25 Estantia, Woodlands Drive
Queenswood, Pretoria, Transvaal(ZA)

(74) Representative: Jenkins, Peter David et al
PAGE, WHITE & FARRER 54 Doughty Street
London WC1N 2LS(GB)

(54) Production of methanol.

(57) A process for producing methanol comprises reacting hydrogen with carbon monoxide and/or carbon dioxide to form a methanol containing product. This product is contacted with a liquid organic absorbent having between 3 and 16 carbon atoms and which is chemically inert with respect to the product, thereby to absorb methanol into the absorbent. Methanol is recovered from the methanol-rich absorbent. The mass proportion of absorbent to methanol recovered is less than 10 mass units absorbent for each mass unit of methanol.

FIG 1

EP 0 430 699 A2

## PRODUCTION OF METHANOL

THIS INVENTION relates to the production of methanol. It relates in particular to a process for producing methanol.

According to the invention, there is provided a process for producing methanol, which comprises reacting hydrogen with carbon monoxide and/or carbon dioxide to form a methanol containing product; contacting the product with a liquid organic absorbent having between 3 and 16 carbon atoms and which is chemically inert with respect to the product, thereby to absorb methanol into the absorbent; and recovering methanol from the methanol-rich absorbent, with the mass proportion of absorbent to methanol recovered being less than 10 mass units absorbent for each mass unit of methanol.

The reaction may be effected by passing an influent gaseous stream containing hydrogen, carbon monoxide and/or carbon dioxide, over a methanol synthesis catalyst at elevated temperature and pressure. The methanol synthesis catalyst may be in the form of a packed bed of catalyst particles or in the form of a slurry of catalyst particles suspended in liquid absorbent. The absorbent will naturally also be selected so as to be chemically inert with respect to the catalyst.

When the catalyst is in the form of a bed of catalyst particles, a single bed of catalyst particles, located in a single reaction vessel, may be provided. However, if desired, a plurality of sequential beds of catalyst particles, each located in a separate reaction vessel, may be provided. The contacting with the liquid absorbent may then take place downstream of the or each catalyst bed, so that the or each catalyst bed constitutes a reaction zone, with the absorption then being effected in a separate absorption zone. Instead, the contacting with the liquid absorbent may take place in the or each catalyst bed, with the or each catalyst bed then constituting both a reaction zone as well as an absorption zone.

In one embodiment of the invention, the absorbent may pass countercurrently through the reaction and absorption zones relative to the influent gaseous stream. In other embodiments of the invention, however, the flows of these two streams may be cocurrent or crossflow. countercurrent flow is, however, preferred when the influent gaseous stream contains substantial concentrations of inert components.

When the catalyst is in the form of a slurry bed suspended in the liquid absorbent, the or each slurry bed may be provided in the lower region of a vessel, with the lower region of the vessel hence constituting both the reaction and the absorption zones. The influent gaseous stream may then be introduced into the vessel at or near its bottom, with methanol-rich absorbent being withdrawn from the slurry bed. In one embodiment of the invention, the process may include separating catalyst particles from the loaded absorbent prior to the loaded absorbent being withdrawn from the vessel. In another embodiment of the invention, the catalyst particle separation may be effected after the loaded absorbent has been withdrawn, with the separated catalyst particles then being recycled or recirculated to the vessel.

The reaction may be effected at a pressure of between 30 and 150 bar, the process including, if necessary, pressurizing the influent gas stream prior to its entering the vessel.

The reaction zone(s) may be maintained at between 180°C and 300°C. This may be effected by inter-cooling between successive reaction zones, by heat removal through tube walls when the catalyst particles are contained in tubes, by feeding cold absorbent to the absorption zone(s), by cooling tubes located in the absorption zone(s), by cooling tubes located in the slurry bed of catalyst particles when the catalyst particles are in the form of a slurry bed, or the like.

The recovery of the methanol from the methanol-rich absorbent may include distilling the methanol-rich absorbent so that methanol and regenerated absorbent are recovered as separate streams.

The process may also include recycling the regenerated absorbent to the absorption zone for reuse. The absorbent recycle ratio, ie the mass proportion of regenerated absorbent to methanol recovered, may be between 0.5 mass units absorbent for each mass unit of methanol, and 9.95 mass units absorbent for each mass unit of methanol, with the regenerated absorbent constituting substantially the entire absorbent introduced into the reaction zone(s), fresh absorbent hence merely being used to make up for losses in the process. Theoretical calculations indicate that the recycle ratio attainable may, however, be as low as 0.1 mass unit absorbent for each mass unit of methanol produced or recovered.

The absorbent may be an alcohol, a paraffinic oil, an olefinic oil, a hydroxylated aromatic or a xylene. In particular, it may be an alcohol having from 3 to 16 carbon atoms eg pentanol, a paraffin having from 7 to 16 carbon atoms such as nonane, an olefin having from 7 to 16 carbon atoms eg 1-heptene, a hydroxylated aromatic having from 6 to 10 carbon atoms eg phenol, a xylene eg 2-xylene, or any mixture of such absorbents.

The invention will now be described by way of example with reference to the accompanying drawings.

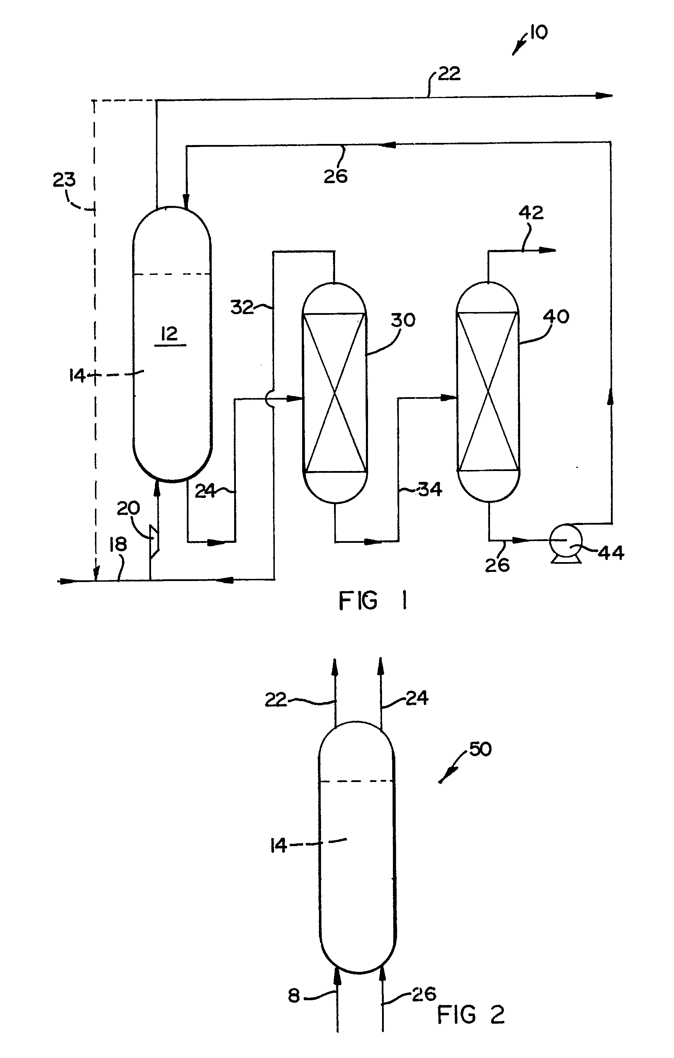

In the drawings, FIGURES 1 to 5 show simplified flow diagrams of processes for producing methanol, according to various embodiments of the invention.

Referring to Figure 1, reference numeral 10 generally indicates a process for producing methanol, according to one embodiment of the invention.

The process 10 includes a vessel 12 containing a packed bed 14 of catalyst particles. The catalyst is a conventional heterogenous catalyst for producing methanol from synthesis gas.

A flow line 18, fitted with a compressor 20, leads into the bottom of the vessel 12. A flow line 22 leads from the top of the vessel 12. A flow line 24 leads from the bottom of the vessel 14, while a flow line 26 leads into the top of the vessel 12.

The process 10 also includes a gas-liquid separation vessel 30, with the flow line 24 leading into the vessel 30. The vessel 30 is of more or less conventional construction for effecting gas-liquid separation. A flow line 32 leads from the top of the vessel 30, and joins the flow line 18 upstream of the compressor 20.

A flow line 34 leads from the bottom of the vessel 30, to a third vessel 40. The vessel 40 is in the form of a distillation column. A flow line 42 leads from the top of the vessel 40, while the flow line 26, fitted with a pump 44, leads from the bottom of the vessel 40.

In use, a gaseous influent or synthesis stream passes along the flow line 18. Typically the influent or feedgas stream comprises hydrogen and carbon monoxide in a mass ratio of about 2:1. Instead of, or in addition to, the carbon monoxide, it may contain carbon dioxide. The feedgas stream can also contain inert gases such as methane or nitrogen. The feedgas stream is compressed, optionally together with some recycle gas via the flow line 32, by the compressor 20 to a pressure of 50-100 bar, at which pressure it enters the vessel 12 so that the vessel 12 is also maintained at about this pressure.

In the catalyst bed 14, exothermic methanol synthesis reactions take place as follows:

(1) $CO + 2H_2 \rightarrow CH_3OH$  $H_{298K} = -90.77$ kJ/mol

(2) $CO_2 + 3H_2$  $CH_3OH + H_2O$  $H_{298K} = -49.52$ kJ/mol

The reaction conditions are selected to obtain a desired balance between degree of conversion and reaction rate, with high temperatures favouring decreased conversions but high reaction rates. However, typically the temperature of the vessel 12 is maintained at between 220°C and 300°C by cooling tubes, etc as hereinbefore described.

Simultaneously, a liquid organic absorbent, typically heptane, enters the vessel along the flow line 26. The absorbent thus trickles over the catalyst particles in the bed 14, countercurrent to the flow of gases up the bed 14. As methanol is produced on the catalyst particles, it is physically absorbed into the absorbent, with little or no chemical reaction between the absorbent and the methanol taking place. Hence, the catalyst bed 14 constitutes both a reaction zone and an absorption zone. Methanol-rich absorbent exits the vessel 12 via the flow line 24, and passes to the recovery zone made up of the vessels 30, 40. Unreacted gases and inerts leave the process via the flow line 22. Optionally, some of this gas can be recycled to the vessel 12 via a flowline 23 indicated in broken line in Figure 1, with the flowline 23 leading into the flowline 18 upstream of the compressor 20.

Naturally, the reaction conditions and absorbent will be selected such that the absorbent remains in liquid phase in the vessel 12.

In the vessel 30, unreacted gases are separated from methanol-rich absorbent, with the unreacted gases being purged, or recycled to the vessel 12 via the flow line 32. The methanol-rich absorbent passes, via the flow line 34, to the distillation vessel 40. In the vessel 40, methanol is distilled off from the absorbent. The methanol exits the vessel 40 via the flow line 42, while regenerated absorbent is recycled, by means of the pump 44 and the flow line 26, back to the vessel 12 for reuse.

Referring to Figure 2, reference numeral 50 generally indicates another form of vessel having a packed bed of catalyst particles.

Flow lines leading into the vessel 50, which are the same or similar to those leading into the vessel 12 of the process 10, are indicated with the same reference numerals.

It will thus be seen that the vessel 50 is substantially the same as the vessel 12, save that the flow of regenerated absorbent entering the vessel 50 along the flow line 26 is cocurrent with the gaseous flow through the vessel 50, rather than countercurrent thereto.

It is further to be understood that methanol can be recovered and the absorbent regenerated in the same recovery zone as that of the process 10.

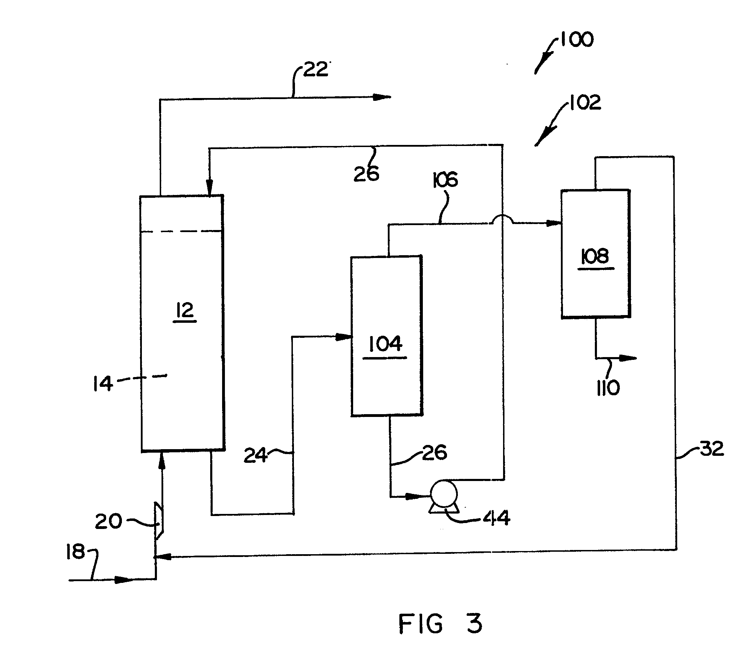

Referring to Figure 3, reference numeral 100 generally indicates a process for producing methanol, according to another embodiment of the invention.

Parts of the process 10 which are the same or similar to those of the process 10, are indicated with the same reference numerals.

The process 100 also includes a vessel 12, with flow lines 18, 26 leading into the vessel, and flow lines 22, 24 leading from the vessel.

The process 100 includes a recovery zone, generally indicated by reference numeral 102. The recovery zone 102 includes a distillation column 104, with the flow line 24 leading into the distillation column 104. In the distillation column 104, unreacted gases and methanol are separated from absorbent, with regenerated absorbent exiting the column 104 at its bottom via the flow line 26, while the methanol and unreacted gases exit the top of the column 104 via a flow line 106. The flow line 106 leads to a gas-liquid separator vessel 108 in which unreacted gases are separated from methanol. The methanol exits the column 108 via a flow line 110, while the unreacted gases leave the top of the column 108 via the flow line 32.

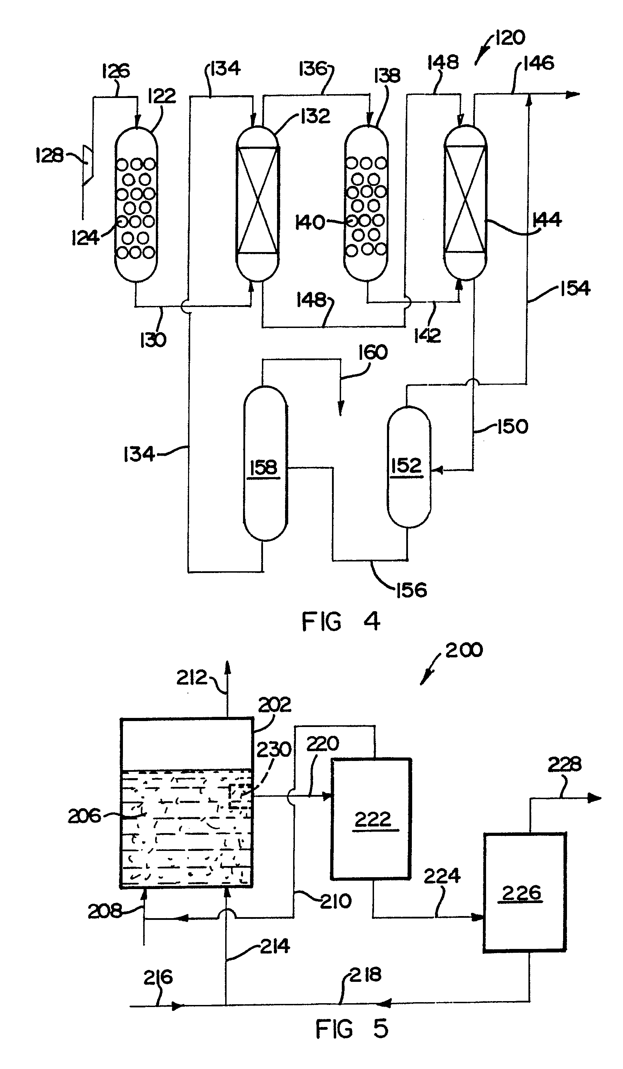

Referring to Figure 4, reference numeral 120 generally indicates a process for producing methanol, according to yet another embodiment of the invention.

The process 120 includes a first reactor vessel 122 containing a packed bed of catalyst particles 124. A feedgas flowline 126, fitted with a compressor 128 leads into the top of the vessel 122, while a methanol-rich gas flowline 130 leads from the bottom of the vessel 122 into the bottom of an absorption vessel 132. A methanol-lean absorbent flowline 134 leads into the top of the vessel 132, while a gas flowline 136 leads from the top of the vessel 132 to the top of a second reactor vessel 138, containing a packed bed 140 of catalyst particles.

A methanol-containing gas flowline 142 leads from the bottom of the vessel 138 to the bottom of a second absorption vessel 144, with an off gas line 146 leading from the top of the vessel 144. A methanol-rich absorbent flowline 148 leads from the bottom of the vessel 132 to the top of the vessel 144, while a methanol-rich absorbent flowline 150 leads from the bottom of the vessel 144 to a degassing unit 152. A desorbed gas flowline 154 leads from the top of the unit 152 to the flowline 146, while an absorbent flowline 156 leads from the bottom of the unit 152 to a methanol/absorbent separator 158. A recovered methanol flowline 160 leads from the top of the separator 158, while the flowline 134 leads from the bottom of the vessel.

In use, feedgas as hereinbefore described enters the top of the vessel 122 at a pressure of 50 to 150 bars. In the vessel 122 at least some conversion of hydrogen and carbon monoxide to methanol takes place, with an accompanying temperature rise. The temperature is controlled as hereinbefore described, so that the gas exiting from the vessel 122 along flowline 130 is typically at a temperature of about 270° C. In the absorber vessel 132, methanol is absorbed from the gas by means of regenerated absorbent, eg heptane, entering along the flowline 134. A drop in temperature, typically to about 220° C, takes place. The gas then passes to the further reactor vessel 138 where further production of methanol takes place, again with an accompanying rise in temperature. The temperature is again typically controlled at about 270° C by heat removal as hereinbefore described. Methanol-rich gas passes from the reactor vessel 138, via the flowline 148, to the further absorber 144. The absorbent from the vessel 132 enters the top of the vessel 144 via the flowline 148. Off gas exits the process via the flowline 146, while methanol laden absorbent passes, via the flowline 150, to the separator 152. Desorbed gas passes via the flowline 154 to the flowline 146, while laden absorbent passes along flowline 156 to the separator 158. Typically, the separator 158 can be a distillation column as hereinbefore described. Regenerated absorbent is recycled via the flowline 134 back to the absorber vessel 132, while methanol is recovered along the flowline 160.

The flow of absorbent and gas through the train of reaction and absorption vessels is cocurrent, and the flows between the vessels, ie the driving force between the vessels, is effected by a decrease in pressure from the one vessel to the next. Only two reaction vessels and two absorption vessels have been shown, but a greater number can naturally easily be provided, if desired.

Referring to Figure 5, reference numeral 200 generally indicates a process for producing methanol, according to still another embodiment of the invention.

The process 200 includes a vessel 202 containing a hold-up or volume 206 of liquid absorbent in which is suspended catalyst particles so that the hold-up 206 is in the form of a slurry. The catalyst particles are kept in suspension by means of the synthesis gas bubbling upwardly through the suspension.

A raw gas flowline 208 enters the bottom of the vessel 202 with a recycled gas flowline 210 leading into the flowline 208. A gas flowline 212 leads from the top of the vessel.

A methanol-lean absorbent flowline 214 leads into the bottom of the vessel 202, with a fresh absorbent make-up flowline 216, as well as a regenerated absorbent flowline 218 leading into the flowline 214. A loaded absorbent flowline 220 leads from the vessel 202 at a higher level, to a separator 222. The flowline 210 leads from the top of the separator 222, while a methanol laden absorbent flowline 224 leads from the bottom of the separator 222 to a further separator 226. A methanol recovery flowline 228 leads from the separator 226, while the flowline 218 leads from the bottom of the separator 226.

In use, the vessel 202 constitutes both a reaction and an absorption zone with methanol, as it is produced by reaction of the gas components in the presence of the catalyst particles, being absorbed into the absorbent which, as hereinbefore described, can be heptane. A liquid exits the vessel 202 along the flowline 220. This liquid consists of a slurry of catalyst particles in absorbent, as well as methanol absorbed into the absorbent. In the vessel 222, gases carried over are separated from the absorbent slurry and returned to the vessel 202 via the flowline 210. The absorbent, still containing catalyst particles, passes to the vessel 226, where methanol is separated from the absorbent and catalyst particles. The methanol is recovered along the flowline 228 while the absorbent as well as the catalyst particles are recycled to the vessel 202 via the flowline 218.

In another embodiment of the invention, a conventional liquid/solids separating means may be provided at the exit of the liquid from the vessel 202, at the location indicated in broken line and reference numeral 230 in Figure 5.

Instead of heptane, any other absorbent as hereinbefore described can be used. The Applicant has derived theoretically the mass of absorbent required per unit mass of methanol absorbed at conditions where methanol is in equilibrium with synthesis gas having a hydrogen to carbon monoxide ratio of 2 to 1, at various temperatures and pressures. This data is set out in Table 1 hereunder. In generating the data for Table 1, the laws for ideal solutions were assumed since little or no experimental data is available for vapour-liquid equilibria of the respective methanol-absorbent mixtures at the specified operating conditions. However, the Applicant has determined experimentally that this approach is sufficiently accurate to identify the absorbents herein specified. For example, it has been found experimentally by the Applicant that, when using pentanol as the methanol absorbent, the ratio of the vapour and liquid phase methanol mole fractions at $230^\circ$C is 0.76, as compared to a theoretical ratio of 0.88. The Applicant believes that this is within the accuracy limits of equipment used, and in any event the experimental error proved to underestimate the methanol solubility.

## TABLE 1

### MASS UNITS ABSORBENT PER MASS UNIT METHANOL RECOVERED

| ABSORBENT | 50 BAR | | | 75 BAR | | | 100 BAR | | |
|---|---|---|---|---|---|---|---|---|---|
| | 230°C | 250°C | 270°C | 230°C | 250°C | 270°C | 230°C | 250°C | 270°C |
| PROPANOL | 18.35 | 509.00 | — | 2.00 | 13.13 | 171.96 | < | 2.82 | 16.47 |
| BUTANOL | 9.13 | 42.67 | 372.11 | 1.72 | 7.40 | 29.0 | < | 2.31 | 8.54 |
| PENTANOL | 7.61 | 24.63 | 95.21 | 1.71 | 6.42 | 19.12 | < | 2.27 | 7.31 |
| HEXANOL | 7.90 | 23.93 | 84.16 | 1.87 | 6.82 | 19.59 | < | 2.49 | 7.86 |
| HEPTANOL | 7.89 | 20.99 | 56.79 | 1.98 | 6.78 | 17.20 | < | 2.59 | 7.60 |
| HEPTANE | 11.18 | 40.17 | 177.77 | 2.22 | 8.60 | 26.81 | < | 2.85 | 9.38 |
| OCTANE | 9.57 | 29.46 | 101.24 | 2.19 | 8.00 | 22.65 | < | 2.87 | 8.97 |
| NONANE | 9.64 | 27.84 | 87.19 | 2.32 | 8.24 | 22.43 | < | 3.04 | 9.32 |
| DECANE | 9.69 | 25.92 | 71.43 | 2.43 | 8.34 | 21.36 | < | 3.18 | 9.39 |
| UNDECANE | 9.98 | 25.26 | 62.69 | 2.58 | 8.58 | 20.82 | < | 3.34 | 9.55 |
| DODECANE | 10.66 | 26.57 | 64.66 | 2.78 | 9.16 | 22.02 | < | 3.59 | 10.20 |
| TRIDECANE | 11.39 | 28.11 | 67.49 | 2.98 | 9.79 | 23.36 | < | 3.86 | 10.89 |
| TETRADECANE | 12.14 | 29.80 | 71.04 | 3.19 | 10.45 | 24.83 | < | 4.13 | 11.62 |
| PENTADECANE | 12.91 | 31.55 | 74.74 | 3.40 | 11.11 | 26.32 | < | 4.40 | 12.37 |
| HEXADECANE | 13.72 | 33.38 | 78.64 | 3.62 | 11.80 | 27.86 | < | 4.68 | 13.12 |
| PHENOL | 6.33 | 16.79 | 46.02 | 1.60 | 5.45 | 13.94 | < | 2.09 | 6.16 |
| RESORCINOL | 6.69 | 16.32 | 38.78 | 1.76 | 5.76 | 13.66 | < | 2.28 | 6.42 |
| PYROGALLOL | 7.61 | 18.53 | 43.34 | 2.01 | 6.56 | 15.43 | < | 2.60 | 7.28 |

LEGEND

'<' means very small

The Applicant believes that the use of the C3-C16 absorbent renders the process for producing methanol according to the invention, particularly effective. For example, an organic absorbent having between 3 and 16 carbon atoms has a high methanol absorption capacity, as indicated in Table 1.

Thus the absorbent, apart from having to be chemically inert or stable, must have as low vapour pressure as possible as this enhances high conversions per pass. However, the higher the molar mass of the absorbent, the lower its degree of methanol absorption. The Applicant thus believes that absorbents having C3-C16 carbon atoms, and in particular the n- forms of these compounds rather than the iso- forms, provide a good balance between vapour pressure and high molar mass. For example, the Applicant believes that with these absorbents, the mass of liquid absorbent required, per unit mass of methanol produced, can be reduced to less than 10 kg/kg, eg about 9 kg/kg or less, while obtaining high conversions of synthesis gas to methanol, per pass of synthesis gas through the reactor vessel(s). Such conversions per pass typically are in the range of 50% to 90%, and can be as high as about 98%, this limitation being set by the solubility of the synthesis or feed gas in the absorbents.

**Claims**

1. A process for producing methanol, characterized in that it comprises reacting hydrogen with carbon monoxide and/or carbon dioxide to form a methanol containing product; contacting the product with a liquid organic absorbent having between 3 and 16 carbon atoms and which is chemically inert with respect to the product, thereby to absorb methanol into the absorbent; and recovering methanol from the methanol-rich absorbent, with the mass proportion of absorbent to methanol recovered being less than 10 mass units absorbent for each mass unit of methanol.

2. A process according to Claim 1, characterized in that the reaction is effected in a reaction zone by passing an influent gaseous stream containing hydrogen, carbon monoxide and/or carbon dioxide, over a methanol synthesis catalyst at elevated temperature and pressure, with the contacting with the absorbent being effected in an absorption zone, the absorbent being selected so as to be chemically inert with respect to the catalyst and comprising an alcohol, a paraffinic oil, an olefinic oil, a hydroxylated aromatic, a xylene, or mixtures thereof.

3. A process according to Claim 2, characterized in that the absorbent comprises an alcohol having from 3 to 16 carbon atoms, a paraffin ahving from 7 to 16 carbon atoms, an olefin having from 7 to 16 carbon atoms, a hydroxylated aromatic having from 6 to 10 carbon atoms, a xylene, or mixtures thereof.

4. A process according to Claim 2 or Claim 3, characterized in that the recovery of the methanol from the methanol-rich absorbent includes distilling the methanol-rich absorbent so that methanol and regenerated absorbent are recovered as separate streams, with the regenerated absorbent being recycled to the absorption zone for reuse.

5. A process according to Claim 4, characterized in that the absorbent recycle ratio is between 0.5 mass units absorbent for each mass unit of methanol, and 9.95 mass units absorbent for each mass unit of methanol, with the regenerated absorbent constituting substantially the entire absorbent introduced into the reaction zone(s), fresh absorbent thus merely being used to make up for losses in the process.

6. A process according to any one of Claims 2 to 5 inclusive, characterized in that the reaction is effected at a pressure of between 30 and 150 bar, and a temperature of between 180° C and 300° C.

7. A process according to any one of Claims 2 to 6 inclusive, characterized in that the catalyst is in the form of a slurry of catalyst particles suspended in liquid absorbent, with the slurry being provided as a slurry bed in the lower region of the vessel and this lower region constituting both the reaction and absorption zones.

8. A process according to any one of Claims 2 to 6 inclusive, characterized in that in the catalyst is in the form of a packed bed of catalyst particles, with the contacting with the liquid absorbent taking place in the catalyst bed so that the catalyst constitutes both the reaction zone and the absorption zone.

FIG 1

FIG 2

FIG 3

FIG 4

FIG 5